# EUROPEAN PATENT APPLICATION

(11) **EP 0 593 789 A1**
(43) Date of publication of application: **27.04.1994**
(21) Application number: 93911978.0
(22) Date of filing: 07.05.1993
(51) Int. Cl.: C12Q 1/68, C12Q 1/70, C12N 15/00, C12N 15/51

(54) **METHOD OF JUDGING PRE-C MUTATION OF HEPATITIS B VIRUS**

(30) Priority: 08.05.1992 JP 116293/92
(71) Applicant: SUMITOMO METAL INDUSTRIES, LTD., Osaka-Shi Osaka 541 (JP)
(72) Inventor: KOSHIZAKA, Takuya 701, 5-7-13 Sagamigaoka, Kanagawa 228 (JP); OKAMOTO, Hiroaki 3311-158 Oaza-Yakushiji, Kawachi-gun Tochigi 329-04 (JP)
(74) Representative: TER MEER - MÜLLER - STEINMEISTER & PARTNER
(86) International application number: JP9300602
(87) International publication number: WO9323567

(57) **Abstract**

A method of judging pre-C mutation of hepatitis B virus accurately in a short time by a simplified operation, which method comprises designing a primer set by introducing a mismatching base in the vicinity of a pre-C mutation site of a hepatitis B virus genome DNA in such a manner as to make it possible to amplify a given region containing the pre-C mutation site and to give a restriction enzyme cleavage pattern which varies according to whether or not the mutation has occured in the amplified DNA, using the primer set to amplify the DNA by the polymerase chain reaction, and inspecting the pattern of cleavage by the restriction enzyme specific for the mutation, thus judging the pre-C mutation of hepatitis B virus or determining the percentage of the viruses thus mutated.

## Description

### Technical Field

The present invention relates to a process for detecting the presence or absence of one or more mutations within the precore (pre-C) region of hepatitis B viral genomic DNA, which is useful for predicting the prognosis of acute hepatitis B. The present invention also relates to a kit for use in the process.

### Background Art

Hepatitis Be (HBe) antigen is observed in the circulatory system of individuals infected with hepatitis B viruses (HBV) in the initial phase of infection. However, during persistent infection, the HBe antigen gradually disappears and HBe antibody is then detected (seroconversion). It has been elucidated that this phenomenon results from the fact that during persistent infection, the wild type HBV which produces the initial infection gradually decreases in number and HBV mutants are produced which are incapable of secreting HBe antigen due to mutations within the pre-C region increasingly in persistent carriers. It is also known that HBV mutants incapable of secreting of HBe antigen have a stronger tendency to induce fulminant hepatitis B than does wild type HBV.

There is no known marker on the HBV viruses which is capable of predicting the prognosis of acute hepatitis B clinically. However, it is believed that predicting the prognosis of acute hepatitis B may be possible by analyzing the sequence of the pre-C region of the viral genome.

HBV mutants which are incapable of secreting HBe antigen have a point mutation from guanine (G) to adenine (A) at nucleotide 83 in the pre-C region. As a result, the codon encoding amino acid 28 is converted from TGG (Trp) to TAG (stop), which inhibits the synthesis of the precursor protein of HBe antigen and prevents secretion of HBe antigen. Furthermore, the mutants found in relatively severe cases of chronic hepatitis B accompanied by viremia often have point mutations from G to A at both nucleotides 83 and 86. In the past, detection of mutations in HBV has been carried out by amplifying the portion containing the mutation site of the DNA by means of the polymerase chain reaction (PCR) technique, linking this DNA fragment to an appropriate plasmid for cloning, isolating and purifying this recombinant, and sequencing. (See Y. Kosaka et al., Gastroenterology, Vol. 100, 1087-1094, 1991; H. Okamoto et al., J. Virology, Vol. 64, 1298-1303, 1990; H. Okamoto, Jikken Igaku, vol. 9, 1263-1268, 1991; Y. Akahane et al., Rinshouigaku, Vol. 16, 586-589, 1990)

However, this detection process takes much time and labor because of the required cloning and sequencing procedures and therefore is not suitable for treating many samples. Moreover, scores of clones have to be assayed simultaneously in order to determine the proportion of mutation when wild type HBV and mutants thereof coexist, and thus, the above process is not practical.

Accordingly, the object of the present invention is to solve the above-described problems of prior art methods, and to provide a process for detecting mutations in HBV and a kit for the detection of HBV mutations, which facilitate fast and precise detection of the mutations by simple procedures.

### Disclosure of Invention

The present inventors have found that the detection of HBV pre-C mutants can be effected with greater ease and accuracy by using newly devised primers for amplifying the region which contains the mutation site within the pre-C region of the HBV genome by means of the mutation site within PCR technique.

The present invention provides a process for detecting one or more mutations within a pre-C region of hepatitis B viral genomic DNA in a sample which comprises hepatitis B viruses, comprising the steps of:
(a) treating the sample to amplify a targeted region which contains a targeted mutation site within the pre-C region of hepatitis B viral genomic DNA by polymerase chain reaction using one or more primer sets, each of said primer sets being capable of amplification of the targeted region and including a primer which contains at least one intentionally introduced base mismatch corresponding to nucleotide(s) near the mutation site to be detected, said base mismatch resulting in the creation of a restriction site which enables amplified DNA products which contain the mutation to be distinguished from those which do not using a restriction enzyme which recognizes said restriction site;
(b) treating the amplified DNA, which contains the base mismatch, with at least one restriction enzyme which recognizes the created restriction site contained in the amplified DNA;
(c) fractionating the treated DNA or its fragments and identifying the treated DNA or its fragments; and
(d) detecting the presence of hepatitis B viruses which contain the targeted pre-C mutation(s) and/ or determining the proportion of the mutants which contains the mutation(s) by analyzing the digestion patterns obtained from the treatment with the restriction enzyme.

A primer set used in step (a) of the above process preferably comprises a first primer and a second primer. The first primer comprises a nucleotide sequence which corresponds to a DNA sequence near the pre-C mutation site, but which does not include the mutation site, and which primer contains at least one intentionally introduced base mismatch which results in the creation of a restriction site and thereby enables the mutation to be detected by treating the amplified DNA with one or more restriction enzymes which recognize the created site. The second primer comprises a nucleotide sequence which, together with the first primer, is capable of providing for amplification of the targeted DNA region which contains the mutation site or site(s).

One or more mutations in the HBV pre-C region can be detected according to the present invention, e.g., the mutation at nucleotides 83 and/or 86.

The present invention also relates to primer sets which are to be used in the above process, and further to kits suitable for detecting the mutations of HBV comprising this primer set and at least one restriction enzyme.

According to the present invention, HBV mutants can be detected. Additionally, the relative proportion of the mutant of HBV viruses, that is the proportion of HBV viruses which contain mutation can be also performed readily and precisely in short time.

### Brief Description of Drawings

Fig. 1 is a schematic representation of a process for detection of the pre-C 83 mutation of HBV;
Fig. 2 is a schematic representation of a process for detection of the pre-C 86 mutation of HBV;

### Best mode for Carrying Out the Invention

The procedure for detection according to the present invention includes the following steps:
(1) amplifying a selected region of the HBV genome which contains one or more mutation site(s) by means of the polymerase chain reaction (PCR) technique using a primer set which is designed for detecting the mutations based on the difference in patterns of digestion of the amplified product with a restriction enzyme(s);
(2) treating the amplified DNA with a restriction enzyme(s) and fractionating and identifying the thus treated DNA or its fragments by appropriate means such as electrophoresis;
(3) analyzing the digestion patterns obtained by digestion with a restriction enzyme(s); and
(4) detecting the mutations based on the restriction patterns or determining the ratio of wild type HBV to HBV mutants.

The first step of this process comprises the amplification of the targeted region of the HBV genome which contains the mutations. Samples suitable for use in detecting such mutations include samples containing HBV prepared from hepatitis B patient's blood, serum, plasma, lymphocyte, tissues and the like. The amplification procedure may be carried out by the commonly used PCR technique, or a two stage PCR technique (nested PCR technique) can alternatively be employed. The two stage PCR technique is one in which in the first stage, the amplification is carried out using a primer set capable of amplification of a longer portion than a target region, and in the second stage the amplification is carried out using a primer set capable of amplification of the target region within the portion amplified in the first stage.

The present process uses a primer sequence which contains at least one base mismatch in the vicinity of the mutation site which results in the creation of a restriction site specific to a selected mutation in the amplified DNA obtained using the mismatch primer. In the case of the two stage PCR technique, the primer which contains at least one base mismatch is utilized in the second stage.

In nature, no restriction site (restriction enzyme recognition site) is created which can distinguish the mutant from the wild type strain, when the G to A mutation at nucleotide 83 or 86 in the HBV pre-C region occurs. However, it is possible to create such a restriction site in this region by designing a primer containing at least one base mismatch. This is because a mismatch primer which only involves a few base mimatches, such as 1-5 mismatches, does not inhibit the amplification of the DNA when it is used in a PCR technique.

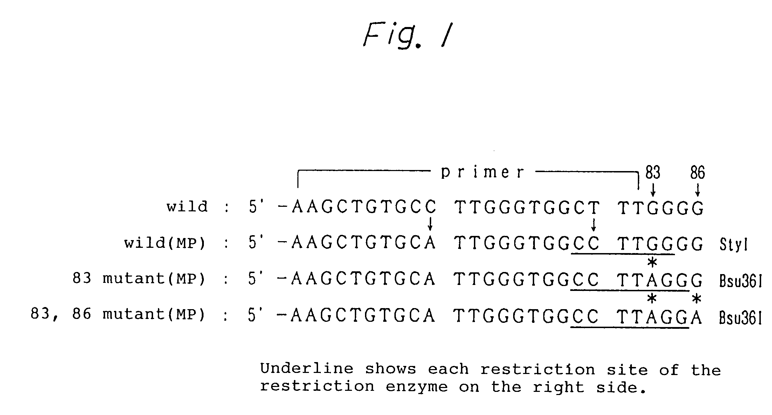

For example, as shown in Fig.1, a mismatch primer (MP) (on a sense chain) having a T to C change at nucleotide, hereinafter nt, 80 is used for the amplification to detect the mutation at nt 83. After amplification using this primer as a first primer, the amplified product has a new restriction site in the region including the mutation site. As a result, a StyI restriction site (CCWWGG) is created in the case of wild strains, and a Bsu36I restriction site (CCTNAGG) is created in the case of 83 mutants. Thus, by amplifying the DNA sequence by PCR using the primer having 22 nucleotides upstream from nt 82 which contains a mismatch nucleotide at nt 80 as the first primer of the primer set, and then digesting the amplified product respectively with StyI or Bsu36I, or with both StyI and Bsu36I and identifying the resulting fragments by means of electrophoresis_{,} wild strains and 83 mutants can be distinguished. Preferably, the primer is designed to further contain a C to A change at nt 70 so that the StyI restriction site which is created within the sequence between the first primer and the second primer may not be digested.

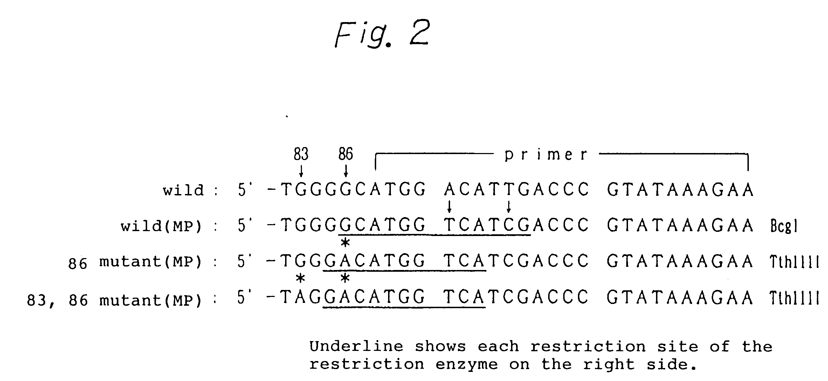

For detection of the mutation at nt 86, a mismatch primer (on an antisense strand) containing an A to T change at nt 92 and a T to C change at nt 96 is used (the expression of nucleotide in Fig.2 is based on the sense strand), and thereby a new restriction site can be created in the wild strains and the mutants, respectively. Thus, a BcgI restriction site (10/12(N)GCA(N)6TCG(N)12/10) occurs in the wild strains and a Tth111I restriction site (GACNNNGTC) occurs in the 86 mutants. Therefore, the wild strains and the 86 mutants can be distinguished by digesting the product amplified by the PCR procedure using the primer having 24 nucleotides downstream from nt 88 which contains mismatch nucleotides at nt 92 and 96 as the first primer, with BcgI or Tth111I, respectively, or with both BcgI and Tth111I, and analyzing digested fragments by electrophoresis.

The second primer used along with the above first primer bearing the mismatch(s) in PCR procedure is one capable of producing along with the first primer amplified DNA which is detectable under appropriate conditions such as electrophoresis. This primer will only typically contain the introduced restriction site which enables detection of the mutation(s). However, other restriction sites may be present if they do not inhibit the detection of mutation(s). The desired amplified DNA can be obtained by the PCR technique using the primer set comprising the first and the second primer. Specifically, in Example 1 a first primer containing a mismatch for detection of 83 mutants and a second primer (on the antisense) containing the sequence downstream from the mutation site of HBV genome are used in the PCR procedure to produce a DNA 99 base pairs long, and the detection of 83 mutants in pre-C region is carried out by restriction enzyme treatment.

When the primer containing the T to C change at nt 80 is used for detection of 83 mutation, a DdeI restriction site (CTNAG) is also created in the case of 83 mutants. Thus, any other restriction enzyme suitable for detection of the mutation may be used. In designing the primer, the DNA sequence of the primer having a mismatch(es) for detection of mutation may be on a sense strand or an antisense strand of the DNA. Moreover, the object of the present invention can be also attained by designing a mismatch primer so that it can produce an amplified DNA having a single restriction site either in wild strains or in mutants, although in the above examples one restriction site is introduced in wild strains and another site is in the mutants. For example, when mismatch primer 5'-AAGCTGTGCCTTGGCCTGCTTT-3'(SIQ ID No.6) (G to C change at nt 75, T to C at nt 76, g to T at nt 77) is used for detection of 83 mutation, EcoNI restriction site (CCTNNNNNAGG) is created only in 83 mutants, and therefore DNA which can be digested with EcoNI is contained in the mutants.
Preferred primer sets used in the present invention are shown below. These primers may be synthesized by known methods, e.g., by using automatic DNA synthesizers.

### Primer Set for Detection of pre-C 83 Mutation:

5'-AAGCTGTGCATTGGGTGGCCTT-3'(PC83F)(SEQ ID NO 1),
5'-AAAGAATTCAGAAGGCAAAAAAGA-3'(PC2)(SEQ ID NO 2);
5'-AAGCTGTGCATTGGGTGGCCTT-3'(PC83F)(SEQ ID NO 1),
5'-GGAGACTCTAAGGCCTCCCGA-3'(PC83R)(SEQ ID NO 3)

### Primer Set for Detection of pre-C 86 Mutation:

5'-TTCTTTATACGGGTCGATGACCAT-3'(PC86R)(SEQ ID NO 4),
5'-GGAGGCTGTAGGCATAAATTGGT-3'(PC86F2)(SEQ ID NO 5).

The amplification by means of the PCR technique may be carried out conventionally by denaturing DNA, annealing the above primers to this, adding heat-stable polymerase and dNTP (dATP, dGTP, dCTP, dTTP) and extending the DNA chain. This repeated cycle of denaturing, primer annealing and DNA chain extension results in amplification of the desired target region.

After amplifying DNA by the PCR technique, the resulting product is treated with one or more restriction enzymes. The selected restriction enzyme will produce different digestion patterns depending on whether the mutation in the pre-C region is present or not. For example, in Example 1, StyI is employed as the restriction enzyme which enzyme is capable of digesting the amplified wild strain DNA product but is incapable of digesting the 83 mutant DNA, and Bsu36I is employed as the restriction enzyme which is capable of digesting the 83 mutant DNA but is incapable of digesting the wild strain DNA product. Also, in Example 3, BcgI is used as the restriction enzyme which enzyme is capable of digesting the wild strain DNA product but is incapable of digesting the 86 mutant DNA, and Tth111I is used as the restriction enzyme which is capable of digesting the 86 mutant DNA product but is incapable of digesting the wild strain DNA products. Such restriction enzymes may be obtained from commercial sources, and DNA digestion procedure may be carried out based on their appended instructions.

The DNA's which have been treated with particular restriction enzyme(s) will then be fractionated under appropriate fractionating conditions such as electrophoresis and then identified. Such procedures may be carried out by conventional means, for example, by 10% polyacrylamide gel electrophoresis, staining with ethidium bromide, and exposure by UV radiation so as to display DNA bands.

DNA fragments which are fractionated and detected are identified based on whether digestion with the restriction enzyme(s) occurs or does not occur. When the restriction enzyme capable of digestion in the case of the wild strains and incapable of digestion in the case of the mutants is used, treated DNA fragments provide a band which is derived from digested fragments if only wild strains exist in the sample. By contrast, if only mutants exist, a band which is derived from original, amplified product is found without cleaving after digestion with the restriction enzyme. If wild strains and mutants coexist, both a band derived from digested product and a band derived from original product are observed, and the intensity of each band indicates the ratio between them. When the restriction enzyme capable of digestion in the case of the mutants and incapable of digestion in the case of wild strains is used, opposite results are obtained. Therefore, use of two different kinds of restriction enzymes can prevent incorrect determination attribute to a decrease in activity of the restriction enzyme and thus enable more precise evaluation. However, relatively accurate results can still be obtained even using a single restriction enzyme.

If a sample contains mutations both at nt 83 and nt 86 in the HBV pre-C region, the presence or absence of each mutation can be detected by amplifying the sample DNA using the primer for detection of 83 mutation or the primer for detection of 86 mutation, respectively, and treating each product with a particular restriction enzyme.

A kit for detection of HBV pre-C mutation according to the present process contains a specific primer set for amplifying the region covering a pre-C mutation site, and the restriction enzyme(s) capable of producing different digestion pattern depending on the presence or absence of the mutation(s).

In determining the type of mutation using this kit, appended data for determination which show the digestion patterns depending on the type of mutation may be used. To estimate the ratio of wild strains and mutants, the intensity of the electrophoresis band after treating with the restriction enzyme may be analyzed visually with the naked eye. For more quantitative results, the electrophoresis pattern may be analyzed by a data analysis instrument such as a commercially available CCD camera.

### Example

The following examples illustrate various embodiments of the invention and are not intended to be limiting in any respect.

20 µl of serum sample containing hepatitis B viruses were mixed with 20 µl of 0.2N solution of NaOH and incubated at 37°C for 1 hour. Then, the mixture was neutralized by adding 20 µl of 0.2N solution of HCl, and centrifuged at 10,000 x g for 5 minutes. The resulting supernatant was obtained as a sample solution of HBV DNA. Using this solution sample, detection of 83 mutation in the HBV pre-C region was carried out and detection of 86 mutation in the HBV pre-C region was carried in Example 3 by means of the procedure described below.

### Example 1

### Detection of 83 mutation in HBV pre-C region (1)

Pre-C 83 mutants were detected by the PCR technique as described below using the above-mentioned DNA sample solution. The primer designed for detection of the mutation in nucleotide sequence on the viral genome was 5'-AAGCTGTGCATTGGGTGGCCTT-3'(PC83F)(SEQ ID NO 1), and the primer for producing 99 base pairs long DNA after amplification was 5'-AAAGAATTCAGAAGGCAAAAAAGA-3'(PC2)(SEQ ID NO 2). These primers, 20 µl of DNA sample solution, 2.5 units of heat-stable DNA polymerase, 10 mM Tris-HCl(pH 8.0), 50 mM KCl, 2.5 mM MgCl₂, 0.01% gelatin, 0.02% Nonidet P-40, and dNTP(dATP, dCTP, dGTP, dTTP)(each final concentration: 200 µM) were mixed and the final volume was adjusted to 100 µl. Then, 50 µl of mineral oil were overlaid and the reaction was allowed to proceed. The amplification of DNA was carried out by 30 repeated cycles of denaturing (at 94°C for 30 seconds), annealing (at 55°C, 1 minute), DNA extension (at 72°C, 1 minute), and further DNA extension (at 72°C, 10 minutes).

About 200ng(10 µl) of the resulting amplified DNA were treated with a restriction enzyme, StyI or Bsu36I. According to a commonly used procedure, the resulting product was subjected to electrophoresis using 10% polyacrylamide gel, stained with ethidium bromide and exposed to UV light, resulting in the display of bands. Evaluation was made using the data shown in Table 1 which describes the fragment size of the band to be detected. When the product was digested only with StyI, there was no mutation in the pre-C region of HBV existing in the serum, and therefore all these viruses were of wild type. When the product was digested only with Bsu36I, all viruses existing in the serum were pre-C 83 mutants. If both coexisted, the intensity of the 99 bp band which resulted from no digestion with each restriction enzyme varied depending on the ratio of wild strains and mutants. The results are shown in Table 2.

**Table 1**

| Data for determination | | |
|---|---|---|
| | ① StyI | ② Bsu36I |
| 83 wild (G) | 80,19 | 99 |
| 83 mutant (A) | 99 | 79,20 |

**Table 2**

| Sample No. | Presence or absence of band (strong ∼ weak ++∼+) | | | | Determination |
|---|---|---|---|---|---|
| | Styl | | Bsu36I | | |
| | 99 bp | 88 bp | 99 bp | 79 bp | |
| 1 | ++ | | | ++ | 83 mutant |
| 2 | + | | | + | 83 mutant |
| 3 | | + | + | | 83 wild |
| 4 | ++ | + | + | ++ | 83 wild, mutant |
| 5 | | ++ | ++ | | 83 wild |
| 6 | + | | | + | 83 mutant |
| 7 | + | + | + | + | 83 wild, mutant |

### Example 2

### Detection of 83 mutation in HBV pre-C region (2)

Pre-C 83 mutants were detected by the PCR technique as described below using the above-mentioned DNA sample solution. The primer designed for detection of the mutation in the nucleotide sequence on the viral genome was 5'-AAGCTGTGCATTGGGTGGCCTT-3'(PC83F)(SEQ ID NO 1), and the primer for producing 161 base pairs long DNA after amplification was 5'-GGAGACTCTAAGGCCTCCCGA-3'(PC83R)(SEQ ID NO 3). These primers, 20 µl of DNA sample solution, 2.5 units of heat-stable DNA polymerase, 10 mM Tris-HCl(pH 8.0), 50 mM KCl, 2.5 mM MgCl₂, 0.01% gelatin, 0.02% Nonidet P-40, dNTP(dATP, dCTP, dGTP, dTTP)(each final concentration: 200 µM) were mixed and the final volume was adjusted to 100 µl. Then, 50 µl of mineral oil were overlaid and the reaction was allowed to proceed. The amplification of DNA was carried out by 30 repeated cycles of denaturing (at 94°C for 30 seconds), annealing (at 55°C, 1 minute), DNA extension (at 72°C, 1 minute), and further DNA extension (at 72°C, 10 minutes).

About 200ng(10 µl) of the resulting amplified DNA were treated with a restriction enzyme, StyI or Bsu36I. According to a commonly used procedure, the resulting product was subjected to electrophoresis using 10% polyacrylamide gel, stained with ethidium bromide, and exposed to UV light, resulting in the display of bands. Analyzing the digestion pattern, evaluation was made using the data shown in Table 3. When the product was digested only with StyI, there was not mutation in the pre-C region of HBV existing in the serum, and therefore, all these viruses were of wild type. When the product was digested only with Bsu36I, all viruses existing in the serum were pre-C 83 mutants. If both coexisted, the intensity of the 161 bp band which resulted from no digestion with each restriction enzyme varied depending on the ratio of wild strains and mutants. The results are shown in Table 4.

**Table 3**

| Data for determination | | |
|---|---|---|
| | ① StyI | ② Bsu36I |
| 83 wild (G) | 142,19 | 161 |
| 83 mutant (A) | 161 | 141,20 |

**Table 4**

| Sample No. | Presence or absence of band (strong ∼ weak ; ++∼+) | | | | Determination |
|---|---|---|---|---|---|
| | Styl | | Bsu36I | | |
| | 161 bp | 142 bp | 161 bp | 141 bp | |
| 1 | ++ | | | ++ | 83 mutant |
| 2 | + | | | + | 83 mutant |
| 3 | | + | + | | 83 wild |
| 4 | ++ | + | + | ++ | 83 wild, mutant |
| 5 | | ++ | ++ | | 83 wild |
| 6 | + | | | + | 83 mutant |
| 7 | + | + | + | + | 83 wild, mutant |

### Example 3

### Detection of 83 mutation in HBV pre-C region (3)

Pre-C 83 mutants was detected by the Nested-PCR technique as described below using the above-mentioned DNA sample solution. 5'-AATGTCAACGACCGACCTTG-3'($012) and 5'-GGAAAGAAGTCAGAAGGCAA-3'($030) were used as the primers of the first stage. These primers, 5 µl of DNA sample solution, 1.25 units of heat-stable DNA polymerase, 10 mM Tris-HCl(pH 8.3), 50 mM KCl, 1.5 mM MgCl₂, 0.001% gelatin, and dNTP(dATP, dCTP, dGTP, dTTP)(each final concentration: 200 µM) were mixed and the final volume was adjusted to 50 µl. Then, 25 µl of mineral oil were overlaid and the reaction was allowed to proceed. The amplification of DNA was carried out by 35 repeated cycles of denaturing (at 94°C, 1 minute), annealing (at 55°C, 1 minute), DNA extension (at 72°C, 1 minute and 30 seconds), and further DNA extension (at 72°C, 7 minutes).

The primer designed for detection of the mutation in nucleotide sequence on the viral genome was 5'-AAGCTGTGCATTGGGTGGCCTT-3'(PC83F), and the primer for producing 99 base pairs long DNA after amplification was 5'-AAAGAATTCAGAAGGCAAAAAAGA-3'(PC2). These primers, 1 µl of the resulting PCR solution of the first stage, 1.25 units of heat-stable DNA polymerase, 10 mM Tris-HCl(pH 8.3), 50 mM KCl, 1.5 mM MgCl₂, 0.001% gelatin, and dNTP(dATP, dCTP, dGTP, dTTP)(each final concentration: 200 µM) were mixed and the final volume was adjusted to 50 µl. Then, 25 µl of mineral oil were overlaid and the PCR of the second stage was allowed to proceed. The amplification of DNA was carried out by 25 repeated cycles of denaturing (at 94°C for 1 minute), annealing (at 55°C, 1 minute), DNA extension (at 72°C, 1 minute and 30 seconds), and further DNA extension (at 72°C, 7 minutes).

About 200ng(10 µl) of the resulting amplified DNA was treated with a restriction enzyme, StyI or Bsu36I. According to a commonly used procedure, the resulting product was subjected to electrophoresis using 10% polyacrylamide gel, stained with ethidium bromide, and exposed to UV light, resulting in the display of bands. Analyzing the digestion patterns, evaluation was made using the data in Table 1. When the product was digested only with StyI, there was no mutation in the pre-C region of HBV existing in the serum, and therefore all these viruses were of wild type. When the product was digested only with Bsu36I, all viruses existing in the serum were pre-C 83 mutants. When both of these viruses coexist, the intensity of the 99 bp band which results after treatment with each restriction enzyme varies dependent on the ratio of the wild strains to the mutants. The same results as shown in Table 2 were obtained.

### Example 4

### Detection of 86 mutation in HBV pre-C region

Pre-C 86 mutants were detected by the PCR technique as described below using the above-mentioned DNA sample solution. The primer designed for detection of the mutation in nucleotide sequence on the viral genome was 5'-TTCTTTATACGGGTCGATGACCAT-3'(PC86R)(SEQ ID NO 4), and the primer for producing 149 base pairs long DNA after amplification was 5'-GGAGGCTGTAGGCATAAATTGGT-3'(PC86F2)(SEQ ID NO 5). These primers, 20 µl of DNA sample solution, 2.5 units of heat-stable DNA polymerase, 10 mM Tris-HCl(pH 8.0), 50 mM KCl, 2.5 mM MgCl₂, 0.01% gelatin, 0.02% Nonidet P-40, and dNTP(dATP, dCTP, dGTP, dTTP)(each final concentration: 200 µM) were mixed and the final volume was adjusted to 100 µl. Then, 50 µl of mineral oil were overlaid and the reaction was allowed to proceed. Amplification of DNA was carried out by 30 repeated cycles of denaturing (at 94°C for 30 seconds), annealing (at 55°C, 1 minute), DNA extension (at 72°C, 1 minute), and further DNA extension (at 72°C, 10 minutes).

About 200ng(10 µl) of the resulting amplified DNA were treated with a restriction enzyme, BcgI or Tth111I. According to a commonly used procedure, the resulting product was subjected to electrophoresis using 10% polyacrylamide gel, stained with ethidium bromide and exposed to UV light, resulting in the display of bands. Analyzing the digestion patterns, evaluation was made using the data shown in Table 5. When the product was digested only with BcgI, there was no mutation in pre-C region of HBV existing in the serum, and therefore, all these viruses were of wild type. When the product was digested only with Tth111I, all viruses existing in the serum were pre-C 86 mutants. If both coexisted, the intensity of 146 bp band which resulted from no digestion with each restriction enzyme varied depending on the ratio of wild strains and mutants. The results are shown in Table 6.

**Table 5**

| Data for determination | | |
|---|---|---|
| | ① BcgI | ② Tth111I |
| 86 wild (G) | 113,34,2 | 149 |
| 86 mutant (A) | 149 | 126,23 |

**Table 6**

| Sample No. | Presence or absence of band (strong ∼ weak ; ++∼+) | | | | Determination |
|---|---|---|---|---|---|
| | BcgI | | Tth111I | | |
| | 149 bp | 113 bp | 149 bp | 126 bp | |
| 1 | + | | | + | 86 mutant |
| 2 | | + | + | | 86 wild |
| 3 | | ++ | ++ | | 86 wild |
| 4 | ++ | + | + | ++ | 86 wild, mutant |
| 5 | | ++ | ++ | | 86 wild |
| 6 | | + | + | | 86 wild |
| 7 | | + | + | | 86 wild |

### Industrial Applicability

According to the present invention, mutation(s) in the HBV pre-C region can be detected by analyzing the digestion pattern obtained from amplified DNA with particular primers. Therefore, the present invention provides a much improved method for detecting mutation(s) readily and accurately in a short time. Furthermore, the process is clinically applicable to samples in which wild strains and mutants coexist, and enables their relative ratios to be determined. Thus, the subject invention provides a convenient method for predicting the prognosis of acute hepatitis B infection based on the detection for the mutation(s) in the HBV pre-C region.

Sequence Listing

SEQUENCE IDENTIFIER NUMBER: 1
SEQUENCE LENGTH: 22
SEQUENCE TYPE: nucleic acid
STRANDEDNESS: single
TOPOLOGY: linear
MOLECULE TYPE: other nucleic acid synthetic DNA
SEQUENCE
AAGCTGTGCA TTGGGTGGCC TT 22

SEQUENCE IDENTIFIER NUMBER: 2
SEQUENCE LENGTH: 24
SEQUENCE TYPE: nucleic acid
STRANDEDNESS: single
TOPOLOGY: linear
MOLECULE TYPE: other nucleic acid synthetic DNA
SEQUENCE
AAAGAATTCA GAAGGCAAAA AAGA 24

SEQUENCE IDENTIFIER NUMBER: 3
SEQUENCE LENGTH: 21
SEQUENCE TYPE: nucleic acid
STRANDEDNESS: single
TOPOLOGY: linear
MOLECULE TYPE: other nucleic acid synthetic DNA
SEQUENCE
GGAGACTCTA AGGCCTCCCG A 21

SEQUENCE IDENTIFIER NUMBER: 4
SEQUENCE LENGTH: 24
SEQUENCE TYPE: nucleic acid
STRANDEDNESS: single
TOPOLOGY: linear
MOLECULE TYPE: other nucleic acid synthetic DNA
SEQUENCE
TTCTTTATAC GGGTCGATGA CCAT 24

SEQUENCE IDENTIFIER NUMBER: 5
SEQUENCE LENGTH: 23
SEQUENCE TYPE: nucleic acid
STRANDEDNESS: single
TOPOLOGY: linear
MOLECULE TYPE: other nucleic acid synthetic DNA
SEQUENCE
GGAGGCTGTA GGCATAAATT GGT 23

SEQUENCE IDENTIFIER NUMBER: 6
SEQUENCE LENGTH: 22
SEQUENCE TYPE: nucleic acid
STRANDEDNESS: single
TOPOLOGY: linear
MOLECULE TYPE: other nucleic acid synthetic DNA
SEQUENCE
AAGCTGTGCC TTGGCCTGCT TT 22

## Claims

1. A process for detecting one or more mutations within a pre-C region of hepatitis B viral genomic DNA in a sample which comprises hepatitis B viruses, comprising the steps of:
(a) treating the sample to amplify a targeted region which contains a targeted mutation site within the pre-C region of hepatitis B viral genomic DNA by polymerase chain reaction using one or more primer sets, each of said primer sets being capable of amplification of the targeted region and including a primer which contains at least one intentionally introduced base mismatch corresponding to the nucleotide(s) near the mutation site to be detected, said base mismatch resulting in the creation of a restriction site which enables amplified DNA products which contain the mutation to be distinguished from those which do not using a restriction enzyme which recognizes said restriction site;
(b) treating the amplified DNA which contains the base mismatch with at least one restriction enzyme which recognizes the created restriction site contained in the amplified DNA;
(c) fractionating the treated DNA or its fragments and identifying the treated DNA or its fragments; and
(d) detecting the presence of hepatitis B viruses which contain the targeted pre-C mutation(s) and/or determining the proportion of the mutants which contain the mutation(s) by analyzing the digestion patterns obtained from the treatment with the restriction enzyme.

2. The process according to Claim 1, wherein the primer set used in step (a) comprises a first primer and a second primer, (i)the first primer having a nucleotide sequence which corresponds to a DNA sequence near the pre-C mutation site, but which does not include the mutation site, and which primer contains at least one intentionally introduced base mismatch which results in the creation of a restriction site and thereby enables the mutation to be detected by treating the amplified DNA with one or more restriction enzymes, (ii)the second primer having a nucleotide sequence which, together with the first primer, is capable of providing for amplification of the targeted DNA region which contains the mutation site or sites.

3. The process according to Claim 2, wherein one or more mutation sites in the pre-C region of hepatitis B viral genomic DNA are detected.

4. The process according to Claim 3, wherein the mutation is a point mutation at nucleotide 83 and/or a point mutation at nucleotide 86 in the pre-C region of hepatitis B viral genomic DNA.

5. The process according to Claim 4, wherein the point mutation at nucleotide 83 in the pre-C region is detected using a primer containing a base mismatch corresponding to nucleotide 80 in the pre-C region.

6. The process according to Claim 4, wherein the point mutation at nucleotide 86 in the pre-C region is detected using a primer containing base mismatches corresponding to nucleotides 92 and 96 in the pre-C region.

7. A primer set suitable for detecting targeted mutation(s) in a pre-C region of hepatitis B viral genomic DNA, which primer set is capable of amplifying a targeted region containing a pre-C mutation site(s) of hepatitis B viral genomic DNA, and which includes a primer containing at least one intentionally introduced base mismatch corresponding to nucleotide(s) near the targeted mutation site, said base mismatch resulting in the creation of a restriction site which enables amplified DNA products which contain the targeted mutation to be distinguished from those which do not using a restriction enzyme which recognizes the created restriction site.

8. The primer set according to Claim 7 comprising a first primer and a second primer, (i)the first primer having a nucleotide sequence corresponding to a DNA sequence near the pre-C mutation site, but does not include the mutation site, and which primer contains at least one intentionally introduced base mismatch which results in the creation of a restriction site and thereby enables the mutation to be detected by treating the amplified DNA with one or more restriction enzymes which recognize the created site, (ii)the second primer having a nucleotide sequence which, together with the first primer, is capable of providing for amplification of the targeted DNA region which contains the mutation site or sites.

9. The primer set according to Claim 8, wherein the mutation is a point mutation at nucleotide 83 and/or a point mutation at nucleotide 86 in the pre-C region of hepatitis B viral genomic DNA.

10. The primer set according to Claim 9, which is suitable for the detection of the mutation at nucleotide 83 in the pre-C region and wherein the first primer has the nucleotide sequence shown in SEQ ID No. 1.

11. The primer set according to Claim 9, which is suitable for the detection of the mutation at nucleotide 83 in the pre-C region, and wherein the first primer has the nucleotide sequence shown in SEQ ID No. 6.

12. The primer set according to Claim 9, which is suitable for the detection of the mutation at nucleotide 86 in the pre-C region, and wherein the first primer has the nucleotide sequence shown in SEQ ID No. 4.

13. A kit suitable for the detection of one or more targeted mutation(s) contained in the pre-C region of hepatitis B viral genomic DNA, comprising the primer set according to any of Claims 7 - 12 and further comprising at least one restriction enzyme which recognizes the created restriction site and which enables the detection of DNA's which comprise the targeted mutation(s).
